# EUROPÄISCHE PATENTSCHRIFT

(11) **EP 1 882 734 B1**
(45) Veröffentlichungstag und Bekanntmachung des Hinweises auf die Patenterteilung: **05.05.2010**
(21) Anmeldenummer: 07112950.6
(22) Anmeldetag: 23.07.2007
(51) Int. Cl.: C12M 1/107

(54) **Behälter, insbesondere Biogasfermenter**
Containers, in particular biogas fermenters
Récipient, en particulier fermenteur de biogaz

(30) Priorität: 26.07.2006 DE 102006035227
(43) Veröffentlichungstag der Anmeldung: 30.01.2008
(73) Patentinhaber: MT-Energie GmbH & Co. KG, 27404 Rockstedt (DE)
(72) Erfinder: Martens, Christoph, 27404, Rockstedt (DE)
(74) Vertreter: von Ahsen, Erwin-Detlef

(56) Entgegenhaltungen:
- EP-A- 0 521 302
- WO-A-98/28402
- DE-A1- 19 633 440
- DE-U1-202005 011 689

## Beschreibung

Die Erfindung betrifft einen Behälter, insbesondere einen Biogasfermenter, mit einer umlaufenden, einen Innenraum umschließenden Wand und einer den Innenraum verschließenden Abdeckung, welche an der Wand gehalten ist, wobei an einem Übergangsbereich von der Wand zur Abdeckung ein Dichtungsband vorgesehen ist.

Biogasfermenter werden üblicherweise mit einer zylindrischen Wand aufgebaut, welche einen Innenraum, in welchem später der Gärprozeß stattfindet, umschließt. Dieser Innenraum ist zunächst mittels einer sogenannten Gasspeicherfolie abgedeckt, unterhalb derer sich der eigentliche Biogasspeicher befindet. Die Gasspeicherfolie ist ihrerseits mittels einer Traglufthaube, welche aus einer festen witterungsbeständigen Folie besteht, als Schutz gegen Witterungseinflüsse und äußere mechanische Belastungen abgedeckt. Die Gasspeicherfolie und die Traglufthaube werden in einer außen an der Wand umlaufenden, C-förmigen Schiene mittels eines Klemmschlauchs gehalten. Damit an der Nahtstelle zwischen der Schiene und den Folien einerseits sowie der Schiene und der Wand andererseits kein Biogas entweichen kann, ist in diesem Bereich ein Dichtungsband angeordnet, welches sich zwischen den Folien und der Schiene über die Schiene hinweg bis hin auf den oberen Rand der Wand erstreckt. Diese Dichtung ist selbstklebend ausgebildet und auf diese Weise mit dem oberen Rand der Wand und der Schiene verklebt. Ein Beispiel eines solchen Biogasfermenters wird in DE 19 633 440 A1 offenbart.

Es hat sich gezeigt, daß die Verklebung des Dichtungsbandes mit der Wand nicht ausreichend ist. Das Biogas unterwandert das Dichtungsband und beschädigt den Kleber, so daß sich das Dichtungsband löst.

Hiervon ausgehend liegt der Erfindung das Problem zugrunde, einen Behälter der eingangs genannten Art derart weiterzubilden, daß das Dichtungsband sicher mit wenigstens der Wand verklebt ist.

Zur Lösung dieses Problems ist der erfindungsgemäße Behälter dadurch gekennzeichnet, daß das Dichtungsband wenigstens mit der Wand mittels einer gesonderten Dichtungsmasse verklebt ist.

Versuche haben gezeigt, daß die gesonderte Dichtungsmasse hinreichend resistent gegen Biogas ist und auch langfristig eine sichere Verklebung zwischen der Wand und dem Dichtungsband ermöglicht.

Die Versuche haben aber gezeigt, daß in dem Fall, in dem eine sich oberhalb des Substrats, aus welchem das Biogas erzeugt wird, bildender Schaum den Bereich des Dichtungsbandes erreicht, dieses Substrat zu einem Aufquellen der gesonderten Dichtungsmasse führt, was ebenfalls zu Lasten der Dichtigkeit geht. Nach einer Weiterbildung der Erfindung ist deshalb vorgesehen, das Dichtungsband zusätzlich mit der Wand zu verschrauben. Dieses geschieht vorzugsweise, indem auf der Wand abgewandten Seite des Dichtungsbandes eine Leiste vorgesehen ist, mittels derer das Dichtungsband mit der Wand verschraubt ist. Letzteres bewirkt, daß das Dichtungsband vollflächig gegen die Wand und damit auf die gesonderte Dichtungsmasse gedrückt wird. Die Dichtungsmasse ist so mechanisch gegen Aufquellen gesichert und ihre dichtenden Eigenschaften bleiben beibehalten.

Die Erfindung wird nachfolgend anhand eines in der Zeichnung dargestellten Ausführungsbeispiels näher erläutert. In der Zeichnung zeigt:

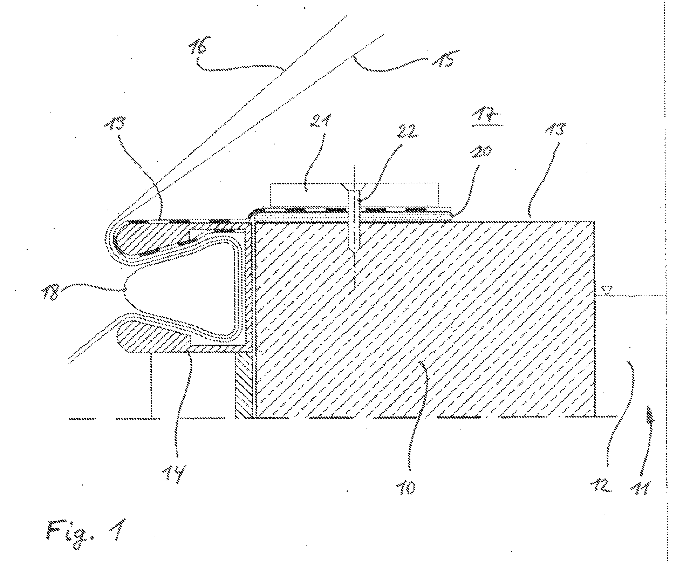

Fig.1 Einen Abschnitt eines Behälters mit den Erfindungsmerkmalen im Vertikalschnitt.

Fig. 1 zeigt einen Abschnitt eines Biogasfermenters, konkret den oberen Bereich einer umlaufenden Wand 10 desselben. Die umlaufende Wand 10 umschließt einen Innenraum 11, in welchem ein Substrat 12 angeordnet ist, aus welchem das Biogas durch vergären desselben erzeugt wird.

Unterhalb des oberen Randes 13 der Wand 10 ist außen eine umlaufende Schiene 14 angeordnet. Diese Schiene 14 ist im Querschnitt C-förmig ausgebildet, wobei die offene Seite in Radialrichtung des Biogasfermenters nach außen zeigt.

Der Innenraum 11 des Biogasfermenters ist zunächst mit einer Gasspeicherfolie 15 abgedeckt. Der Zwischenraum 17 zwischen dem Substrat 12 und der Gasspeicherfolie 15 bildet einen Biogasspeicher für das produzierte Biogas, aus welchem es letztlich herausgeführt wird. Oberhalb der Gasspeicherfolie 15 ist eine Traglufthaube 16 angeordnet, welche aus einer witterungsbeständigen, festen Folie besteht. Die Traglufthaube schützt die Gasspeicherfolie 15 vor Witterungseinflüssen und mechanischer Beschädigung.

Die Gasspeicherfolie 15 und die Abdeckhaube 16 werden mit ihrem äußeren Rand in die C-förmige Schiene wie dargestellt eingelegt und dort mittels eines Klemmschlauchs 18 in an sich bekannter Weise gehalten.

Der Übergangsbereich von den Folien 15, 16 zur Wand 10 wird mittels eines Dichtungsbandes 19 abgedichtet. Der in Radialrichtung des Biogasfermenters gesehen nach außen weisende Bereich des Dichtungsbandes 19 ist innen in die Schiene 15 mit eingelegt und wird hier über die Folien 15, 16 mittels des Klemmschlauchs 18 geklemmt. Das Dichtungsband 19 ist weiterhin über den oberen Bereich der Schiene 14 hinweg bis auf den oberen Rand 13 der Wand 10 geführt und insgesamt an seiner der Schiene 14 bzw. der Wand 10 zugewandten Seite selbstklebend ausgebildet. Mit dem oberen Rand 13 der Wand 10 ist das Dichtungsband 19 zusätzlich mittels einer gesonderten Dichtungsmasse 20 verklebt. Diese Dichtungsmasse 20 ist resistent gegen Biogas. Als geeignet hat sich eine elastische Dichtungsmasse auf Basis eines 1-Komponenten Polyurethan Klebers erwiesen. Das Dichtungsband 19 läuft vollständig auf dem oberen Rand 13 der Wand 10 um.

Um ein Aufquellen der gesonderten Dichtungsmasse 20 auch bei aggressivsten Medien, beispielsweise durch einen sich oberhalb des Substrats 12 bildenden Schaum zu verhindern, ist das Dichtungsband 19 zusätzlich mit der Wand 10 verschraubt. Zu diesem Zweck ist auf das Dichtungsband 19, also auf der dem oberen Rand 13 abgewandten Seite des Dichtungsbandes 19 eine Leiste 21 aufgelegt. Die Leiste 21 läuft ebenfalls vollständig auf dem oberen Rand 13 um, wobei es sich um eine einstückige Leiste oder um eine Leiste aus mehreren (Bogen)Segmenten handeln kann. Diese Leiste ist mittels einer geeigneten Schraube 22 in an sich bekannter Weise mit der Wand 10 verschraubt.

Die Erfindung ist nicht darauf beschränkt, daß das Dichtungsband 19 mit der gesonderten Dichtungsmasse 20 auf dem oberen Rand 13 der Wand 10 angeordnet ist. Vielmehr kann zusätzlich oder alternativ das Dichtungsband 19 mit der Dichtungsmasse 20 bei entsprechend geführter Gasspeícherfolie 25 und/oder Abdeckhaube 16 auch an der Außen- oder Innenseite der Wand 10 angeordnet sein. Insbesondere ist es möglich, zumindest die Abdeckhaube 16 bis über den oberen Rand 13 der Wand 10 hinweg außen an der Wand 10 weiter nach unten, unter Umständen bis zum Fuß derselben (bis zum Boden) zu führen, um auf diese Weise auch die Wand 10 vor Witterungseinflüssen (Betonkorrosion) zu schützen.

### Bezugszeichenliste:

- 10: Wand
- 11: Innenraum
- 12: Substrat
- 13: Rand
- 14: Schiene
- 15: Gasspeicherfolie
- 16: Abdeckhaube
- 17: Biogasspeicher
- 18: Klemmschlauch
- 19: Dichtungsband
- 20: Dichtungsmasse
- 21: Leiste
- 22: Schraube

## Patentansprüche

1. Behälter, insbesondere Biogasfermenter, mit einer umlaufenden, einen Innenraum (11) umschließenden Wand (10) und einer den Innenraum (11) verschließenden Abdeckung (15, 16), welche an der Wand (10) gehalten ist, wobei an einem Übergangsbereich von der Wand (10) zur Abdeckung (15, 16) ein Dichtungsband (19) vorgesehen ist, **dadurch gekennzeichnet, daß** das Dichtungsband (19) wenigstens mit der Wand (10) mittels einer gesonderten Dichtungsmasse (20) verklebt ist.

2. Behälter nach Anspruch 1, **dadurch gekennzeichnet, daß** das Dichtungsband (19) zusätzlich mit der Wand (10) verschraubt ist.

3. Behälter nach Anspruch 2, **dadurch gekennzeichnet, daß** auf einer der Wand (10) abgewandte Seite des Dichtungsbandes (19) eine Leiste (21) vorgesehen ist, mittels derer das Dichtungsband (19) mit der Wand (10) verschraubt ist.

## Claims

1. Container, in particular a biogas fermenter, with a circumferential wall (10) enclosing an inner chamber (11), and a cover (15, 16) held on the wall (10) to seal the inner chamber (11), wherein a sealing tape (19) is provided on a transition zone from the wall (10) to the cover (15, 16), **characterised in that** the sealing tape (19) is adhesively bonded at least to the wall (10) by means of a separate sealing compound (20).

2. Container according to Claim 1 **characterised in that** the sealing tape (19) is additionally screwed to the wall (10).

3. Container according to Claim 2 **characterised in that** on one side of the sealing tape (19) facing away from the wall (10) there is provided a strip (21), by which the sealing tape (19) is screwed to the wall (10).

## Revendications

1. Récipient, en particulier un fermenteur de biogaz, avec une paroi circulaire, un volume intérieur (11) est constitué par de paroi circulaire (10) et une partie de la paroi circulaire avec une couverture de protection (15, 16) sous la forme d'un film fixé à la paroi (10) ou en outre à une plage de transition de la paroi (10) pour assurer à la couverture de protection (15,16) où est prévue un bande d'étanchéité (19) , **caractérisée en ce que** ladite bande d'étanchéité (19) est pourvue d'une masse d'étanchéification spécifique (20) collée à la paroi (10).

2. Récipient selon la revendication 1, **caractérisé en ce que** la bande d'étanchéité (19) est vissée à la paroi (10).

3. Récipient selon la revendication 2, **caractérisé en ce que** la bande d'étanchéité (19) collée à la paroi (10), est prévu avec un liston (21) permettant à la bande d'étanchéité (19) d'être vissée à la paroi (10).
